Europäisches Patentamt

European Patent Office

Office européen des brevets

(19)

(11) Numéro de publication: **0 267 833**
**B1**

(12) **FASCICULE DE BREVET EUROPEEN**

(45) Date de publication du fascicule du brevet: 23.01.91

(51) Int. Cl.⁵: **B 01 J 23/88,** C 07 C 7/163

(21) Numéro de dépôt: 87402355.9

(22) Date de dépôt: 21.10.87

(54) **Perfectionnement au procédé de désulfuration des essences terpéniques.**

(30) Priorité: 29.10.86 FR 8615037

(43) Date de publication de la demande:
18.05.88 Bulletin 88/20

(45) Mention de la délivrance du brevet:
23.01.91 Bulletin 91/04

(84) Etats contractants désignés:
AT ES SE

(56) Documents cités:
DE-A-2 045 490
FR-A-2 361 154
US-A-2 037 781
US-A-3 061 555
US-A-3 312 750
US-A-3 930 984
US-A-3 957 625

(73) Titulaire: SOCIETE NATIONALE ELF AQUITAINE
(PRODUCTION)
Tour Elf, 2, Place de la Coupole, La Défense 6
F-92400 Courbevoie (FR)
(73) Titulaire: SOCIETE DES DERIVES RESINIQUES
ET TERPENIQUES
30 rue Gambetta
F-40300 Dax (FR)

(72) Inventeur: Casbas,Francoise
1 rue Saint-Pierre
64300 Orthez (FR)
Inventeur: Duprez,Daniel
21 bis rue du Touffenet
86000 Poitiers (FR)
Inventeur: Ollivier,Jean
Croix de Buzy
64260 Arudy (FR)
Inventeur: Rolley,Raymond
"Paile"
40560 Vielle Saint-Girons (FR)

(74) Mandataire: Kohn, Armand
5 Avenue Foch
F-92380 Garches (FR)

Courier Press, Leamington Spa, England.

# EP 0 267 833 B1

**Description**

La présente invention se rapport à la purification des essences terpéniques renfermant des impuretés sulfurées; elle s'applique en particulier aux essences qui constituent un sous-produit de la fabrication de la pâte à papier.

L'invention concerne plus spécialement un procédé de purification résidant dans la désulfuration catalytique, par de l'hydrogène, de coupes terpéniques issues de la distillation des essences brutes, impures.

L'industrie papetière est un fournisseur important d'essence de papeterie qui contient surtout les α-pinène, β-pinène, $\Delta^3$ carène, myrcène, dipentène et éventuellement des dérivés moins fréquents, comme camphène et autres. Malheureusement, le procédé de délignification du bois, utilisé pour libérer la cellulose, dit procédé KRAFT, basé sur la solubilisation de la lignine à l'aide d'un mélange de soude et de sulfure de sodium, introduit des quantités notables de polluants soufrés dans l'essence extraite des autoclaves de cuisson de la pâte de bois. Ces polluants sont une gêne importante par l'odeur désagréable qu'ils introduisent d'une part, et par les modifications chimiques et physiques des propriétés des terpènes d'autre part. Il faut donc éliminer ces composés soufrés, afin de retrouver les propriétés des terpènes issus de l'essence de térébenthine dite de gemme. De nombreuses méthodes ont pour cela été mises au point. Les plus utilisées sont basées sur le traitement à l'hypochlorite de sodium. Mais ces traitements présentent plusieurs inconvénients parmi lesquels: opérations lourdes, nécessitant un grand nombre d'étapes qui rendent difficile le traitement continu; ces traitements chimiques classiques introduisent dans les terpènes des produits indésirables, contenant du chlore, ou de l'azote; ces procédés désodorisent, mais ne désulfurent par vraiment, leur efficacité restant limitée.

Pour y remédier on a proposé, en 1963, un procédé de désulfuration de l'α-pinène par un traitement catalytique à l'hydrogène. Ainsi, le brevet US—A—3 312 750 indique-t-il le passage de l'α-pinène en contact avec un catalyseur de molybdate de cobalt sur de l'alumine, à une température de 149° à 260°C, sous une pression d'hydrogène de 0 à 35 bars. Ce procédé s'est révélé bien efficace en ce qu'il permet l'élimination d'environ 66 à 90% du soufre présent dans les terpènes, mais il comporte l'inconvénient de transformer une forte fraction de l'α-pinène; cette altération chimique est due en particulier à l'isomérisation et à la saturation des doubles liaisons des composés traités. Elle conduit à des composés beaucoup moins intéressants pour l'industrie que ne le sont les composants de l'essence de térébenthine non modifiée.

Cette question de transformation est mentionnée dans le brevet US précité, uniquement dans le cas d'expérimentation à hautes pressions, mais, lorsqu'on cherche à reproduire ces opérations, on constate une transformation des produits traités atteignant jusqu'à 55% environ.

Un progrès important a été accompli récemment, dans cette technique, par l'utilisation de certains charbons actifs comme support des catalyseurs au Co et Mo. Avec ce procédé, on peut atteindre une désulfuration par hydrogénation des terpènes de l'ordre de 90%, accompagnée d'une faible transformation, inférieure à 8%, de l'α-pinène, alors que la technique antérieure, notamment celle de l'US précité, affectait plus de 40% d'α-pinène. Ce progrès intéressant comporte cependant la sujétion du choix très restreint du support a en charbon actif, les autres supports courants, tels qu'alumine, silice, silicates d'alumine, etc. ne donnant pas les résultats souhaités.

La présente invention apporte à la technique sus-indiquée un nouveau perfectionnement; elle permet d'utiliser toutes sortes de supports pour le catalyseur à base de $CoO$ et $MoO_3$ et d'obtenir une désulfuration poussée, jointe à une faible modification chimique des terpènes, industriellement acceptable.

Les supports, utilisables pour le catalyseur suivant l'invention, peuvent être les différentes matières inorganiques habituellement employées à cet effet, par exemple silice, alumine, divers silico-aluminates, oxyde de titane, zircone, charbon actif, etc.

L'invention est basée sur l'observation imprévue que l'adjonction d'une substance basique aux catalyseurs Co—Mo, sur différents supports minéraux, réduit considérablement l'activité isomérisante de ces systèmes catalytiques. Il devient ainsi possible d'opérer à des températures assez élevées, pour pousser le plus loin possible la désulfuration, sans modification sensible de la composition.

Ainsi, le catalyseur suivant l'invention, constitué par un support inorganique portant des oxydes de Co et Mo, contenant en outre un composé basique d'un métal alcalin ou/et alcalino-terreux est caractérisé en ce que le rapport molaire $CoO/MoO_3$ dans le catalyseur est de 2 à 5. Ce sont surtout les oxydes, silicates, aluminates et molybdates de tels métaux qui conviennent bien au nouveau catalyseur suivant l'invention.

Les métaux, dont des composés basiques font partie du nouveau catalyseur, sont notamment Na, K, Li, Mg, Ca, Ba, et éventuellement, d'autres alcalins ou/et alcalino-terreux, étant bien entendu que des composés de plusieurs d'entre eux peuvent être présent à la foi dans le catalyseur. Pour des raisons économiques et de facilité, le sodium est d'un emploi particulièrement pratique; il peut être pris, par exemple, sous la forme d'oxyde, d'hydroxyde, de carbonate, de bicarbonate, de silicate, de molybdate, d'aluminate ou de silico-aluminate pour son introduction dans le mélange catalytique. Il en est de même des autres composés alcalins et alcalino-terreux.

Dans le catalyseur perfectionné, suivant l'invention, le composé basique, alcalin ou/et alcalino-terreux, est en une proportion telle que l'ensemble du catalyseur ne présente aucune acidité. Cette proportion dépend donc de la nature du support, à savoir de l'acidité initiale de celui-ci. Ainsi faut-il davantage de composé basique dans le cas d'un support d'alumine, qui habituellement comporte des sites acides, que dans celui de silice pouvant être pratiquement neutre. Une partie du composé basique sert à la neutralisation de l'acidité représentée par MoO₃.

Exprimé par rapport à MoO₃, le nombre d'équivalents de composé basique est généralement de 2 à 3 par mole de MoO₃.

Le procédé de l'invention donne de meilleurs résultats avec un excés de cobalt par rapport au molybdène, de 2,5 à 3,5 pour 1 MoO₃. Il en résulte que les proportions pondérales, dans le nouveau catalyseur sont de préférence 5 à 9% CoO avec respectivement 2,77 à 5,7% MoO₃ et 0,2 à 7% Na₂O, la différence à 100 étant constituée par le support, en particulier: silice, charbon ou alumine.

La préparation du catalyseur s'effectue à la manière connue dans l'art. Le support est imprégné d'une solution aqueuse de sel thermodissociable de chacun des métaux Co et Mo, par exemple nitrate de Co et molybdate d'ammonium; on procède également à l'addition d'un composé basique, alcalin ou/et alcalino-terreux. Ce composé peut d'ailleurs être pris sous laforme d'un molybdate correspondant, par exemple molybdate de Na, K, Li, Ca, Mg ou autre. Le mélange est séché, puis calciné à une température de 450°C à 550°C.

L'hydrodésulfuration des terpènes, avec le catalyseur suivant l'invention, est de préférence effectuée à une température de 160°C à 300°C, et surtout entre 200°C et 280°C, sous une pression relative d'hydrogène de 0 à 5 bars et avec une vitesse spatiale du réactif comprise entre 0,1 et 10h⁻¹. Les rapports molaires préférés H₂/terpènes se rangent entre 1 et 15. Les conditions particulièrement favorables sont: température 250°±5°C; pression 0,1 à 1 bar; vitesse spatiale 0,2 à 0,5 h⁻¹; rapport H₂/terpènes 5 à 8.

L'invention est illustrée non limitativement par les exemples qui suivent:

Exemple 1

On prépare un catalyseur par imprégnation de billes d'une silice de surface spécifique de 250 m²/g présentant un volume poreux de 0,6 ml/g, avec une solution aqueuse de nitrate de cobalt et d'heptamolybdate d'ammonium. Après séchage et calcination de 6 h à 500°C, le catalyseur, dénommé catalyseur A, contient 7% de CoO et 4,4% de MoO₃, en poids.

On prépare un deuxième catalyseur B, à partir de la même silice, mais en opérant en deux imprégnations successives. La première concerne le molybdène introduit sous forme de molybdate de sodium; la deuxième le cobalt, introduit sous forme de nitrate.

Après séchage et calcination à 500°C pendant 6h, on imprègne avec une solution aqueuse de soude de telle manière que le catalyseur final contienne 7% CoO, 4,4% MoO₃ et 2,5% Na₂O en poids, soit 3,05 moles CoO et 1,32 Na₂O par mole de MoO₃; Les deux catalyseurs, A et B, servent à traiter une coupe terpénique de β-pinène. On opère à 200°C, sous la pression atmosphérique avec un volume de catalyseur de 70 ml et une vitesse spatiale du réactif de 0,43 h⁻¹, le rapport molaire H₂/terpène étant de 7.

Le catalyseur A permet de faire passe le taux initial de soufre de 1660 ppm à 80 ppm, ce qui représente une élimination de 95%, mais le taux de transformation est de 85,4% alors qu'avec le catalyseur B, le taux de soufre passe de 1900 ppm à 760 ppm, soit une désulfuration de 60%, pour un taux de transformation de seulement 3%.

Dans un autre essai, similaire, avec le catalyseur B, on a porté la température à 295°C, pour traiter une charge de β-pinène. Le taux de soufre passe alors de 2343 ppm à 115 ppm, soit 95% de désulfuration, pour un taux de transformation de 21,3%, taux qui reste inférieur, même à plus haute température, à celui que donne le catalyseur A.

La récapitulation de ces résultats:

| | % Taux de désulfuration | Transformation |
|---|---|---|
| Catalyseur A (sans soude); 200°C | 95 | 85,4 |
| Catalyseur B (avec soude); 200°C | 60 | 3 |
| Catalyseur B (avec soude); 295°C | 95 | 21 |

montre que la transformation chimique du β-pinène est considérablement diminuée grâce à la présence de substance basique dans le catalyseur (B) supporté par de la silice.

Exemple 2

Des catalyseurs semblables à ceux de l'exemple 1 sont préparés avec cette seule différence que le support est un charbon actif de surface spécifique de 900 m²/g. Les désulfurations du β-pinène sont effectuées dela même façon qu'a l'exemple 1 et — dans le cas de catalyseur D — elles sont appliquées également à de l'α-pinène et à une essence de papeterie.

Voici les résultats obtenus:

| Catalyseur | Soufre ppm | | Désulfur. | Transform. |
| | avant | après | % | % |
| --- | --- | --- | --- | --- |
| AC-sans composé basique | | | | |
| β-pinène | 715 | 270 | 62,2 | 90 |
| D-avec 2,5% Na₂O | | | | |
| β-pinène | 1325 | 262 | 80,2 | 8 |
| α-pinène | 362 | 164 | 54,1 | 9,9 |
| essence: | 2374 | 300 | 87,5 | |
| α-pinène | | | | 1,2 |
| β-pinène | | | | 26,0 |
| Δ³-carène | | | | 0,8 |

Tout comme dans l'exemple 1, on voit ici, grâce au composé basique (catalyseur B), une très forte réduction du taux de transformation chimique des terpènes traités, alors que le support est le charbon actif à la place de la silice.

Exemple 3

On prépare trois catalyseurs E, F et G avec un support de billes d'alumine présentant une surface spécifique de 80 m²/g et un volume poreux total de 0,55 ml/g. Ils contiennent 7% de CoO et 4,4% de MoO₃ en poids.

*Catalyseur E:* est obtenu à partir d'une solution aqueuse de nitrate de cobalt et d'heptamolybdate d'ammonium, sans addition d'aucune substance basique.

*Catalyseur F:* On opère en deux imprégnations successives; la première introduit le molybdène sous la forme de molybdate de sodium; la deuxième le cobalt, pris à l'état de nitrate. Il s'ensuit que ce catalyseur F contient 1 mole Na₂O par mole de MoO₃, soit 1,89% en poids de Na₂O dans le catalyseur fini.

*Catalyseur G:* même mode de préparation que pour le catalyseur F, mais on ajoute encore un supplément de soude, pour que la teneur finale du catalyseur en Na₂O soit de 2,5% en poids.

L'hydrogénation de coupes terpéniques, conduisant à la désulfuration de ces coupes, effectuée sur chacun de ces catalyseurs, avec des vitesses spatiales de 0,2 à 0,43 h⁻¹, sous la pression atmosphérique, à 200°C et 245°C, a conduit aux résultats réunis au Tableau suivant:

| Catalyseur | t°C | Désulfurat. % | Transformat. % |
|---|---|---|---|
| E-sans composé basique | | | |
| Δ³ carène | 200°C | 85,2 | 20 |
| F—1,89% Na₂O | | | |
| Δ³ carène | 200°C | 89,4 | 4,3 |
| β-pinène | 200°C | 39,3 | 10 |
| essence de papeterie: | 200°C | 37,5 | |
| α-pinène | | | 6,1 |
| β-pinène | | | 42,8 |
| Δ³ carène | | | 0 |
| G—2,5% Na₂O | | | |
| Δ³ carène | 200°C | 64 | 0,6 |
| α-pinène | 200°C | 53,4 | 0,7 |
| β-pinène | 200°C | 82,6 | 0,5 |
| essence de papeterie: | 200°C | 70,7 | |
| α-pinène | | | 0,5 |
| β-pinène | | | 0 |
| Δ³ carène | | | 0 |
| α-pinène | 245°C | 86,5 | 0,5 |
| β-pinène | 245°C | 72,7 | 0,8 |
| essence de papeterie: | 245°C | 77,3 | |
| α-pinène | | | 3,9 |
| β-pinène | | | 0 |
| Δ³ carène | | | 0 |

Ainsi donc, l'alumine en tant que support du catalyseur, donne d'excellents résultats et — comme les autres supports des exemples 1 et 2 — permet d'abaisser à très peu de choses la transformation chimique des terpènes. On peur remarquer l'effet très favorable d'une addition de 2,5% de Na₂O (catalyseur G) et la possibilité, qui en résulte, de travailler à température plus élevée; on trouve, en effet, à 245°C, pour l'essence de papeterie, une désulfuration de 77,3% avec seulement 3,9% de transformation du seul α-pinène, ce qui constitue un résultat industriel très convenable.

En poussant la température à 300°C, avec l'essence de papeterie, l'hydrodésulfuration était de 70% et seul l'α-pinène a subi une transformation chimique à raison de 5,1%, ce qui est encore fort acceptable en comparaison de ce que donne la technique antérieure.

**Revendications**

1. Catalyseur perfectionné pour la désulfuration des terpènes provenant de la fabrication du papier, par traitement en phase vapeur par de l'hydrogène, qui comprend des oxydes de cobalt et de molybdène sur un support inorganique, et renferme un composé basique alcalin ou/et alcalino-terreux, caractérisé en ce que le rapport molaire CoO/MoO₃, dans ce catalyseur, est de 2 à 5.

2. Catalyseur suivant la revendication 1, dont la teneur en composé basique est de 2 à 3 équivalents par mole de $MoO_3$.

3. Catalyseur suivant la revendication 1 ou 2, caractérisé en ce que sa teneur pondérale en CoO est de 5 à 9% et celle de $MoO_3$ est de 2,7 5,7%.

4. Catalyseur suivant une des revendications 1 à 3, caractérisé en ce que le rapport molaire $CoO/MoO_3$ est de 2,5 à 3,5.

5. Catalyseur suivant une des revendications précédentes, caractérisé en ce que la totalité de $MoO_3$ s'y trouve sous la forme de molybdate de sodium, $Na_2MoO_4$.

**Patentansprüche**

1. Verbesserter Katalysator für die Entschwefelung von aus der Papierherstellung herrührenden Terpenen durch Behandlung mit Wasserstoff in der Gasphase, welcher Kobalt- und Molybdänoxide auf einem anorganischen Träger umfaßt und eine alkalische und/oder erdalkalische basische Verbindung einschließt, dadurch gekennzeichnet, daß das molare Verhältnis $CoO/MoO_3$ im Katalysator 2 bis 5 beträgt.

2. Katalysator nach Anspruch 1, dessen Gehalt an basischer Verbindung 2 bis 3 Äquivalente pro Mol $MoO_3$ ausmacht.

3. Katalysator nach Anspruch 1 oder 2, dadurch gekennzeichnet, daß sein gewichtsmäßiger Gehalt an CoO 5 bis 9% beträgt und der an $MoO_3$ 2,7 bis 5,7%.

4. Katalysator nach einem der Ansprüche 1 bis 3, dadurch gekennzeichnet, daß das molare Verhältnis $CoO/MoO_3$ 2,5 bis 3,5 ist.

5. Katalysator nach einem der vorstehenden Ansprüche, dadurch gekennzeichnet, daß das gesamte $MoO_3$ darin in Form von Natriummolybdat, $Na_2MoO_4$, vorliegt.

**Claims**

1. Improved catalyst for desulphurizing terpenes, issued from paper manufacture, by a treatment with hydrogen, in vapor phase, which comprises cobalt and molybden oxides on an inorganic support, and contains an alkali or alkaline-earth basic compound, characterized in that the molar ratio $CoO/MoO_3$, in the catalyst, is 2 to 5.

2. Catalyst according to claim 1, whose content in basic compound amounts to 2 to 5 equivalents per mole of $MoO_3$.

3. Catalyst according to claim 1 or 2, characterized in that its content by weight in CoO is 5 to 9% and that in $MoO_3$ is 2.7 to 5.7%.

4. Catalyst according to one of claims 1 to 3, characterized in that the molar ratio $CoO/MoO_3$ is 2.5 to 3.5.

5. Catalyst according to any of preceding claims, characterized in that the totality of $MoO_3$ finds therein in the form of sodium molybdate, $Na_2MoO_4$.